# EUROPEAN PATENT APPLICATION

(11) **EP 1 721 614 A1**
(43) Date of publication of application: **15.11.2006**
(21) Application number: 05460019.2
(22) Date of filing: 07.07.2005
(51) Int. Cl.: A61K 31/704, A61P 31/14

(54) **Epirubicine hydrochloride for treating hepatitis C virus**

(30) Priority: 12.05.2005 PL 37500805
(71) Applicant: Institut National de la Santé et de la Recherche Médicale (INSERM), 69003 Lyon (FR); Instytut Biochemii I Biofizyki, 02-106 Warszawa (PL)
(72) Inventor: Cova, Lucyna, 69003 Lyon (FR); Trepo, Christian, 69500 Bron (FR); Narayan, Ramamurthy, Headington Oxford OX3 8LX (GB); Borowski, Peter, 22179 Hamburg (DE); Kulikowski, Tadeusz, 02-116 Warszawa (PL); Zagorski-Ostoja, Wlodzimierz, 02-665 Warszawa (PL)
(74) Representative: Brodowska, Iwona

(57) **Abstract**

Second medical application of epirubicine hydrochloride as an active substance inhibiting the hepatitis C virus (HCV) replication while having low toxicity in Huh7 cells.
Epirubicine hydrochloride is employed in therapy of HCV infections.
Pharmaceutically accepted forms of the anti-HCV drug, best if applied as water solutions suited for injections, according to the invention, are distinguished by containing epirubicine hydrochloride applied at cumulated, antiviral therapeutic doses comprising 1 - 100 mg/m² of body surface (0,02 - 2 mg; 0,0344 - 3,44 µM per kg of body weight).

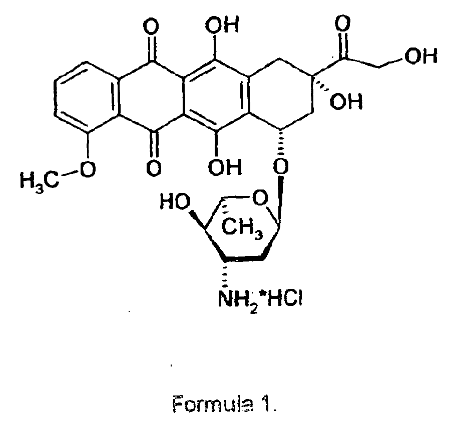

## Description

The present invention relates to new biomedical application of 7-O-(3'-amino-2',3',6'-trideoxy-α,β-L-arabinohexpyranosyl)-adriamycinone hydro-chloride (4'-epidoxorubicin hydrochloride, 4'-epi-adramycin hydrochloride, epirubicine hydrochloride) (Formula 1) and the use of this compound to the treatment of hepatitis C virus (HCV) infections. Hepatitis C virus is of particular importance: it is highly pathogenic member of hepaciviruses of Flaviviridae superfamily, widely distributed overall the world (according to WHO report ca 300 million infected people). Chronic active hepatitis develops in over 85% of acutely infected carriers and leads to liver cirrhosis and hepatocellular carcinoma (Hagedorn and Rice, Eds., The Hepatitis C Viruses, Springer, Heidelberg 2000).

Since the discovery of HCV, numerous trials have been made for anti-HCV chemotherapeutic agents. However, only combination of nucleoside ribavirin with interferon α2b or α2a and/or its pegylated derivatives were approved for the treatment of HCV infections. In addition these therapies were only partially effective (40-50% "cures") and caused a lot of undesired side effects, such as: influenza-like symptoms, headache, hemolysis, nausea, anorexia, anemia, etc. Therefore there is a great need for other anti-HCV agents necessary to control the chronic infection and to reduce progression to hepatocellular carcinoma.

Epirubicine hydrochloride is known as potent antitumor drug with low toxicity and is cell cycle phase nonspecific anthracycline (Arcamone et al., Canadian Patent, 1974, No. 1046507. Its mechanism of action is not fully understood. Epirubicin hydrochloride interacts between base pairs of DNA resulting in inhibition of DNA and RNA synthesis. Interaction causes DNA cleavage by topoisomerase II (Coukell and Faulds, Drugs 1997, 53: 453-82). Epirubicine hydrochloride is also an inhibitor of DNA and RNA helicase activity (Bachur et al., Molecular Pharmacol. 1992, 41: 993-998; Bachur et al., Biochem. Pharmacol. 1998, 55: 1025-1034). The drug is also active against Moloney Sarcoma Virus (MSV) (Arcamone et al., Canadian Patent, 1974, No. 1046507). Epirubicine hydrochloride is toxic to mammalian haemopoietic cells and cardiac tissue, however causes less haemotological and cardiac toxicity than equimolar doses of another widely used anti-tumor drug, doxorubicin (adriamycin). In addition, the iron chelator dexrazoxane reduces epirubicine-induced cardiac damage (Coukell and Faulds, Drugs 1997, 53: 453-82). In antitumor therapy in man maximum cumulative dose of epirubicin hydrochloride is 900 - 1000 mg/m². On the other hand, Kaplan-Meier plot of cumulative probability of developing congestive heart failure in man vs. total cumulative dose for epirubicine points for the "safe" dose of 100 - 150 mg/m² (Launchburry and Habboubit, Cancer Treatment Reviews 1993, 19: 197-228; Praga et al., Clinical Measurement in Drug Evaluation 1991, pp. 131-142).

We have now surprisingly discovered that anthracycline antibiotic, epirubicine hydrochloride (Formula 1) as referred to below has extremely potent inhibitory activity against hepatitis C virus (HCV).

According to the present invention there is provided the use of anthracycline antibiotic epirubicine hydrochloride of Formula 1 or its pharmaceutically acceptable forms for the treatment of HCV infection, especially water solutions to injection.

The compound of Formula 1 and its pharmaceutically acceptable forms are hereinafter referred to as compound according to the invention.

The following example is not intended to limit the scope of the invention in any way.

### Example 1. Antiviral activity and cytotoxicity testing.

The potential antiviral (anti HCV) activity of epirubicin hydrochloride was tested in *in vitro* cell culture model using the HCV subgenomic replicon stably transfected into Huh7 cell lines (clone BM4.5) (Ju-Tao Gao et al., J Virol. 2001*)*.

Huh7 cells containing the HCV replicon were grown in cell culture medium in presence of varying concentration of epirubicin. The media and drug were changed everyday for 5 days. After 5 days of treatment the cells were lysed and total RNA extracted. The isolated RNA was then assayed for presence of HCV replication by Northern Blot analysis, using a HCV probe targeting the NS5B region of HCV. In addition, the hybridisation with the □-actin radiolabelled probe allowed the normalization of the results i.e. to assess the impact of drug on housekeeping gene expression.

The cell lines were treated everyday with epirubicin from concentration of 20 nM to 2 nM for 5 days and at the end of treatment RNA from the treated cells were isolated. The total RNA was then probed with a HCV specific probe using Northern blot analysis to detect the HCV RNA. The resulting blot was then exposed to phosphorimager densitometry and the viral replication quantified.

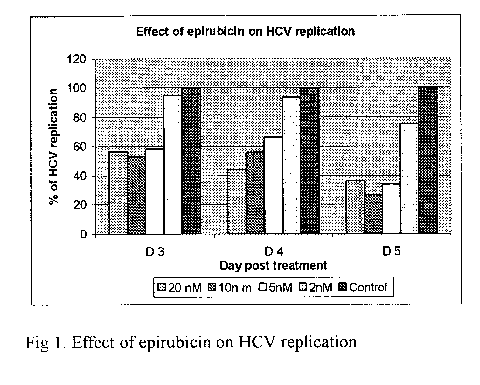

Results of analysis show that epirubicin at concentrations from 20 nM to 5 nM effectively inhibited of HCV replication in the subgenomic replicon system tested in vitro (Figure 1).

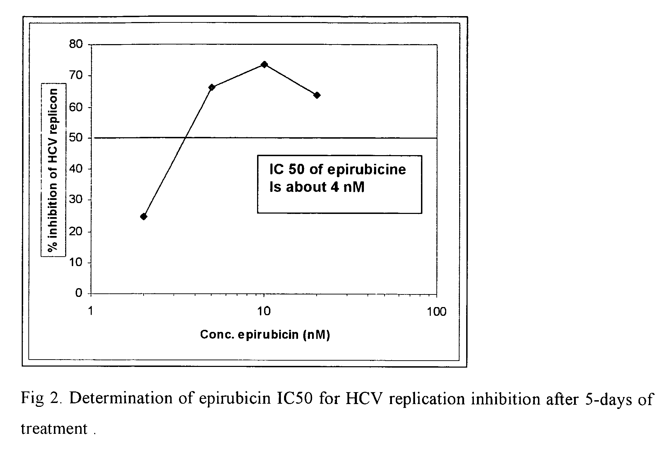

As illustrated in Figure 2, 50% inhibitory concentration (IC₅₀) of epirubicin hydrochloride with regards to the anti-HCV activity measured in HCV replicon system was found to be extremely low since it was of about 3-5 nM, while its 50% cytotoxic concentration (CC₅₀) in Huh7 cells was > 0,1 nM i.e. selectivity index equal to 25.

In this study we show for the first time that epirubicine hydrochloride, a well-known anti-tumour drug is also capable to exert anti-HCV effects at extremely low, nanomolar concentrations and should be regarded as extremely potent drug against HCV infections with some selectivity and lack of toxicity at cumulated antiviral doses equal to 1-100 mg/m² of man (0,02 - 2mg; 0,0344 - 3,44 µM per body weight).

## Claims

1. Second medicinal application of epirubicine hydrochloride (Figure 1), **characterized by** its use as an active substance actively inhibiting hepatitis C virus replication with simultaneus low toxicity against Huh7 cells.

2. Pharmaceutically acceptable forms of the drug against HCV infections, distinguished by containing epirubicine hydrochloride applied at cumulated, antiviral therapeutic doses comprising 1 - 100 mg/m² of body surface (0,02 - 2 mg; 0,0344 - 3,44 µM per kg of body weight).

## Amended claims

### Amended claims in accordance with Rule 86(2) EPC.

**1.** Second medicinal application of epirubicine hydrochloride (Formula 1), **characterized by** its use as an active substance actively inhibiting hepatitis C virus replication with simultaneous low toxicity against Huh7 cells, in maximal cumulated dose of 0.02 to 1.00 mg per kg of body weight.

**2.** Pharmaceutically acceptable forms of the drug against HCV infections, are distinguished by their content of epirubicin hydrochloride as the active substance, applied at very low, nontoxic, antiviral maximal cumulated dose of 0.02 to 1.00 mg per kg of body weight).
